# EUROPEAN PATENT APPLICATION

(11) **EP 1 852 110 A1**
(43) Date of publication of application: **07.11.2007**
(21) Application number: 06008370.6
(22) Date of filing: 24.04.2006
(51) Int. Cl.: A61K 9/20

(54) **Co-precipitate of chitin or chitin derivative and a silicon dioxide or derivative for the use as tablet excipient**

(71) Applicant: The Jordanian Pharmaceutical Manufacturing Co. Ltd., 11710 Naor (JO)
(72) Inventor: Badwan, Adnan, 11185 Amman (JO); Al-Remawi, Mayyas, 13713 Russiefa (JO); Rashid, Iyad Said, 11151 Amman (JO)
(74) Representative: Winkler, Andreas Fritz Ernst

(57) **Abstract**

The invention is related to a co-precipitate comprising at least one hydrophilic polymeric compound selected from the group consisting of chitin, chitin derivatives, other polyglucosamines, or any salts, solvates or mixtures thereof, and at least one water-absorbant compound selected from the group consisting of silicon dioxide, derivatives thereof, or any mixtures thereof, a method of preparing the same and novel uses thereof.

## Description

### Field of Invention

The present invention relates to a novel co-precipitate, a method of preparing the same as well as uses of chitin, chitin derivatives or the novel co-precipitate as a filler and/or a disintegrant for solid pharmaceutical dosage forms.

### Background of the Invention

Chitin is a polyN-acetylglucosamine very abundant in nature. Chitin is present in the exoskeletons of crustaceans, the cuticles of insects and cell walls of most fungi. [E. Guibal, Heterogenous catalysis on chitosan-based materials: a review. Prog. Polym. Sci., 30 (2005) 71-109]

Chitin is a natural substance and highly available in nature. It is inexpensive, non-toxic, biodegradable, biocompatible when compared with other polymers. Chitin is considered to be non-digestible by human when taken by oral route; this is due to lack of specific digestion enzymes, which are present in some bacteria. [K. Oungbho;and B. Muller. Chitosan Sponges as Sustained Release Drug Carriers. Int.J. Pharm.156, pp 229-237, 1997]

Chitosan, the deacetylated chitin derivative, is well known in art. Pharmaceutical uses of chitosan are very numerous. The scientific and medical literature lists hundreds of industrial, medical and dietary applications for chitosan. These include, protection of sensitive drugs from the deactivating enzymes, preparation of artificial cells, using chitosan to trap hemoglobin, promotion of bone repair, bum dressing and contact lens material. As a drug delivery vehicle, it has been examined extensively by the pharmaceutical industry for its potential in controlled drug delivery systems. [J. Karlsen, and O. Skaugrud. Exicipient Properties of Chitosan. Manufacturing Chemist., 62, pp 18, 1991; I Orienti, K. Aieda, C. Ponti, E. Gianasi, V. Zecchi. Progesterone Loaded Chitosan Microspheres. Effect of triethylene glycol glutarate linked to the chitosan molecule on drug release. S.T.P. Pharm. Sci., 6, pp 424-429, 1996; H. Takeuchi, H. Yamamoto, T. Niwa; T. Hino and Y. Kawashima. Internal absorption of insulin in rats from mucoadhesive chitosan-coated liposomes. Pharm. Res.13, pp 896-901, 1996]

In order to prepare a solid dosage form, many additives are used in addition to the active materials.

The first group of additives is necessary for maintaining the physical structure of the solid dosage form before the patient intake. These include diluents, binders, and lubricants. The second group of added substances helps to give additional desirable physical characteristics to the finished tablet. Included in this group are colors, and in the case of chewable tablets, flavors, sweetening agents, and coating materials.

An important additive that plays its role after ingestion of the solid dosage form is called the disintegrant. It is a substance, or a mixture of substances, added to a tablet to facilitate its breakup or disintegration after administration. The active ingredient must be released from the tablet matrix as efficiently as possible to allow for a rapid dissolution. Materials serving as disintegrants have been chemically classified as starches, clays, celluloses, algins, or gums.

The most popular disintegrants are corn and potato starch which have been well-dried and powdered.

In addition to the starches a large variety of materials have been used and are reported to be effective as disintegrants. This group includes Veegum HV, Ac-Di-Sol, methylcellulose, agar, bentonite, cellulose and wood products, natural sponge, cation-exchange resins (e.g., Amberlite IRP-88), alginic acid, guar gum, citrus pulp, and carboxymethylcellulose. [D. Gissinger and A. Stamm. Drug Develop. Ind. Pharm., 6, 511 (1980), "A Comparative Evaluation of the Properties of Some Tablet Disintegrants"; E. M. Rudnic, C. T. Rhodes, J. F. Bavitz, and J. B. Schwartz, Drug Develop. Ind. Pharm., 7, 347 (1981), "Some Effects of Relatively Low Levels of Eight Tablet Disintegrants on a Direct Compression System"; H. A. Lieberman and L. Lachman, Pharmaceutical Dosage Forms: Tablets, Volume 1, pages 139-140; and R. E. Gordon, The Thermodynamic Characterization of the Solid Surface Interaction for Three Tablet Disintegrating Agents, Ph.D. Thesis, Purdue University, 1981].

Another important additive playing a role during tablet formation is called filler/binder or excipient needed for bond formation. It is a substance added to facilitate formation of a hard tablet upon compaction of the powder formula especially with poorly compactable active materials. Fillers usually have powerful flow and compaction properties such as microcristalline cellulose.

In our invention, a novel composition excipient of chitin or its derivatives and silica plays the role of the disintegrant and filler at the same time.

### Summary of the Invention

The invention is related to a co-precipitate comprising at least one hydrophilic polymeric compound selected from the group consisting of chitin, chitin derivatives, other polyglucosamines, or salts, solvates or mixtures thereof, and at least one water-absorbable compound selected from the group consisting of silicone dioxide, derivatives thereof or any mixtures thereof.

Moreover, the invention is concerned with a method for preparing a co-precipitate of the invention by mixing a wet mass of the hydrophilic polymeric compound with a suspension or solution of the water-absorbable compound in an appropriate ratio and adjusting the process conditions in order to allow the co-precipitate to form.

Finally, the invention is related to the use of a compound selected from the group consisting of chitin, chitin derivatives or a co-precipitate according to the invention as a filler and/or disintegrant for solid pharmaceutical dosage forms.

It also provides a compactable mixture that does not effect the tablet physical properties.

### Figures

Figure 1: DSC thermograms of diclofenac K, chitin silica, and a physical mixture of chitin silica:diclofenac K 1:1 w/w
Figure 2: DSC thermograms of pseudoephedrine HCl, chitin silica, and a physical mixture of chitin silica pseudoephedrine HCl 1:1 w/w
Figure 3: Powder X-ray Diffraction of silica and chitin silica co-precipitate
Figure 4: Powder X-ray Diffraction of chitin and chitin silica co-precipitate

### Detailed Description of the Invention

Chitin or its derivatives or related compounds co-processed with silicon dioxide or related compounds have been found to have a superior disintegration activity. Chitin is a hydrophilic polymer that is water-insoluble, but still can absorb water. This character gives chitin or its derivative a disintegration power. However, the substance has a bad flow and compression properties. The co-precipitation with silicon dioxide or related water-absorbant compounds provides the polymer with proper flow and compression properties, and increases the power of polymer for water absorption

When chitin or its derivatives utilizes a colloidal silicon dioxide, it has been found that the resultant excipient product surprisingly provides disintegration power which is substantially improved even in comparison to normal commercially available disintegrants. Another surprising effect is the powerful improvement in flow and compaction of the new co-precipitate excipient compared to each component when taken alone.

The optimal composition contains silicon dioxide in the range of 1-75% w/w, and the most preferable concentration is about 50% w/w.

Chitin or a chitin derivative such as chitosan, or related compound, in the form of a wet mass is mixed with suspension or solution of silicon dioxide or related compound. The resultant co-precipitate is substantially water-insoluble. Therefore, there is no appreciable dissolution of either ingredient in the aqueous slurry. After a uniform mixture of the ingredients is obtained in a wet mass, the wet mass is dried to provide chitin-silica based excipient particles.

Chitin and its derivatives, as materials for use herein, have an average degree of deacetylation of from 0-50% and preferably 0-10 %.

Chitin or chitosan materials may generally have a wide range of molecular weights that are suitable for use in the present invention, typically such materials for use herein have a molecular weight ranging from 1,000 to 10,000,000 gram per moles and more preferably 2,000 to 1,000,000 and most preferably 50,000-1,000,000.

The pH of the mixture of the compounds of the co-precipitate of the invention depends on the preparation method. The preferred pH is 4-10, typically in the range of 4 to 7.

Particularly suitable polyglucosamines for use herein include aminopolysaccharides salts, especially chitin and chitosan salts including organic and inorganic salts known in the art.

Particularly suitable modified chitin for use herein includes water-soluble or water-insoluble covalently bonded chitin or chitosan derivatives or ionically bonded chitin or chitosan derivatives known in the art.

According to the present invention the articles comprise as an essential component a water-absorbant compound, such as silicon dioxide, a derivative thereof (such as silicate) or a mixture thereof.

Silicon dioxide exists in a variety of crystalline forms and amorphous modifications, any of which are suitable for the use herein. In particular, silicon dioxide having a high surface area is preferred such as colloidal silica.

According to the present invention the articles typically comprise from 5 to 300gm⁻² more preferably from 10-250 gm⁻², most preferably from 15 to 200 gm⁻² of silica.

Optional agents such as fillers, lubricants, fat absorbing materials, pH-buffering agents may be included to facilitate and/or improve coprecipitate properties.

Methods of co-precipitation may include any industrial technique known in the art such as spray drying, freeze drying or simple solution mixing.

Chitin silica is a superdisintegrating filler product which is mainly presented in solid form. It can be used as filler and disintegrant in immediate release solid dosage forms. Silicated chitin or chitosan when compressed, using any compression machine known in the art, forms a strong compact. This compact exhibits a superdisintegration power when exposed to aqueous medium. Therefore, the novel excipient is regarded as a superdisintegrating filler.

### Examples

### Example 1

### Physical properties (flowability and compressibility) of powder and compact of chitin silica

General method for preparation of chitin silica and its physical characterization:
Chitin was suspended in acidic medium (for example hydrochloric acid, acetic acid, phosphoric acid, etc). The suspension was added stepwise with vigorous stirring to either silica suspension prepared by suspending silica of similar chitin weights in basic medium (for example sodium hydroxide, potassium hydroxide, etc.), or silica solution of similar weights as chitin. The mixture's pH was adjusted to pH values between 6-7. The product was filtered out, washed with distilled water, and dried in the oven to complete dryness prior to sieving to the desired particle size distribution. The bulk and tap density of the dried powder of chitin silica was determined as shown in Table 1.
Chitin silica powder was compressed using Manesty Single Punch Machine (U.K.) using different compression forces. Punch was circular with a diameter of 13 mm. The tablet weight was fixed at 470 mg. Tablet hardness was measured using Hardness tester (Pharmatest, Germany) and tablet disintegration was determined using a known disintegration apparatus (Pharmatest, Germany). Results are also summarized in Table 1.

**Table 1: Summary of physical characteristic of chitin silica powder and compact**

| Physical property | chitin silica | chitin | | |
|---|---|---|---|---|
| Bulk density (g/ml) | 0.420 | 0.19 | | |
| Tap density | 0.500 | 0.23 | | |
| Compressibility % | 16 | 17.4 | | |

| Compression Force (KN) | Hardness (N) Chitin silica | Hardness (N) Chitin | Disintegration time (Sec) Chitin Silica | Disintegration time (Sec) Chitin |
|---|---|---|---|---|
| 30 | 62 | NA | 8 | NA |
| 35 | 81 | NA | 10 | NA |
| 40 | 92 | 9 | 15 | NA |
| 45 | 105 | 25 | 25 | NA |

| | | | | |
|---|---|---|---|---|
| *NA: not applicable | | | | |

The advantages of coprecipitation of silica on chitin can be summarized as follows:
Chitin silica resulted in a denser grade than chitin. This enables more die filling in compression machines. Flow properties of chitin silica can be considered good to excellent. The chitin silica showed compactability over a wide range of compression forces, whereas this was not shown to the same extent by chitin.

### Example 2

### The use of chitin silica as a filler and disintegrant

Pseudoephedrine HCl was used as a model drug due to its poor compactability.
The drug was mixed with chitin silica at different ratios, the tablet weight was fixed at 240mg, and the applied pressure of the hydraulic press was fixed at 1500 kg. Results are shown in Table 2.
The results indicate that chitin silica is considered a suitable filler which increases the tablet strength or hardness when a poor compactable drug is incorporated in the matrix tablet especially when the drug percentage is below 50%.

**Table 2: Summary of tablets hardness and disintegration time for different mixtures of pseudoephedrine HCl and chitin silica**

| **% Drug** | **% Chitin-Silica** | **Hardness (N)** | **Disintegration Time (seconds)** |
|---|---|---|---|
| 100 | 0 | Friable | ---- |
| 90 | 10 | Friable | ---- |
| 50 | 50 | 50 | 60 |
| 30 | 70 | 110 | 60 |
| 10 | 90 | 167 | 50 |
| 0 | 100 | 237 | 90 |

### Example 3

### Adsorption of active materials to the chitin silica excipient

The model drugs selected for adsorption are diclofenac potassium representing acidic drugs and pseudoephedrine HCl representing basic drugs. Around 50 milligrams of diclofenac potassium or pseudoephidrine HCl were dissolved in 50ml of deionised water, the chitin-silica polymer was added to the drug solutions at the weight ratios of drug to polymer: 1:1, 1:2, 1:10. The samples were left on the shaker for 24 hours together with standards and blanks for each drug. Results are shown in Tables 3 and 4. The assay was almost constant which indicates that no adsorption isotherm for either acidic or basic drug occurred i.e. chitin silica is an inert and compatible excipient for most of active ingredients of similar behavior.

**Table 3: Adsorption effect of diclofenca potassium at different chitin-silica ratios.**

| **Sample** | **Drug weight (mg)** | **Polymer Weight (mg)** | **Final conc. (mg/100ml)** | **%Assay** | **% Interference** |
|---|---|---|---|---|---|
| 1:1 Drug:polymer | 50.3 | 50.3 | 1.28 | 99.7% | |
| 1:2 Drug:polymer | 50.1 | 100 | 1.28 | 101.6% | |
| 1:10 Drug:polymer | 57.8 | 578 | 1.3872 | 99.9% | |
| Blank | 0 | 500 | 12.0* | ----- | 1.4% |

| | | | | | |
|---|---|---|---|---|---|
| *Concentration of the polymer only. | | | | | |

**Table 4: Adsorption effect of pseudoephedrine HCl at different chitin-silica ratios.**

| **Sample** | **Drug weight (mg)** | **Final conc. (mg/100ml)** | **%Assay** | **% Interference** |
|---|---|---|---|---|
| 1:2 Drug:polymer | 55.8 | 17.856 | 101.2% | |
| 1:10 Drug:polymer | 58.2 | 18.624 | 101.1% | |
| Blank | 500 | 160 | ----- | 1.3% |

Tables 3 and 4 show that chitin silica has no adsorption characteristics over acidic and basic drugs.

### Example 4:

### Chemical compatibility of drugs with chitin silica

The prepared chitin-silica powder was mixed with two drugs: pseudoephedrine HCl (model basic drug) and diclofenac potassium (model acidic drug) at a ratio of 1:1 w/w mixtures. The samples were analyzed using differential scanning calorimeter DSC (heating rate 10 °C/min).

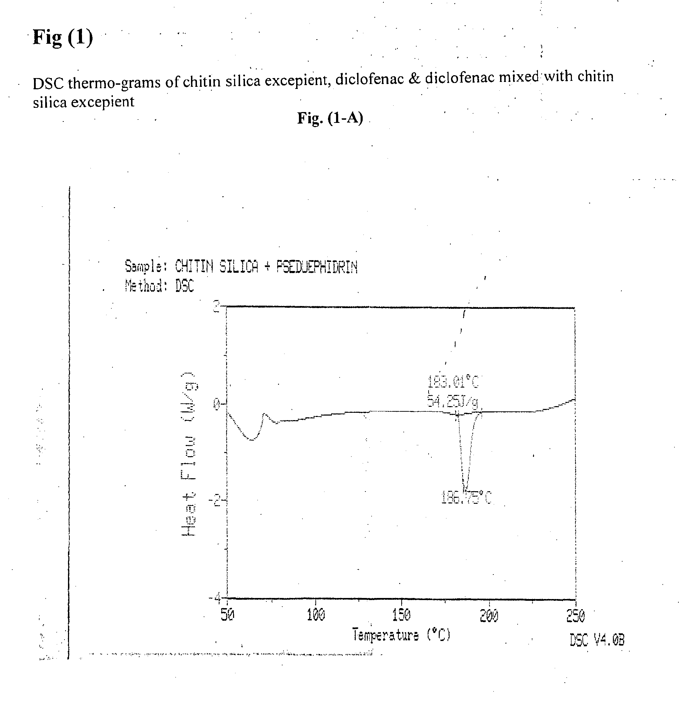

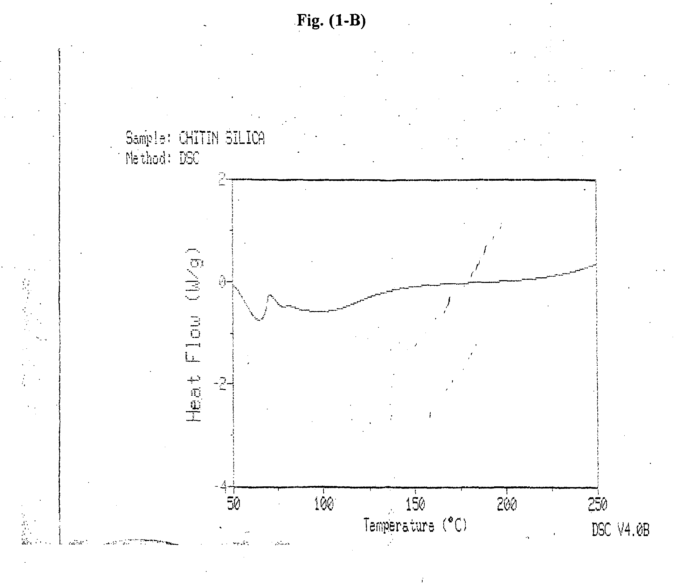

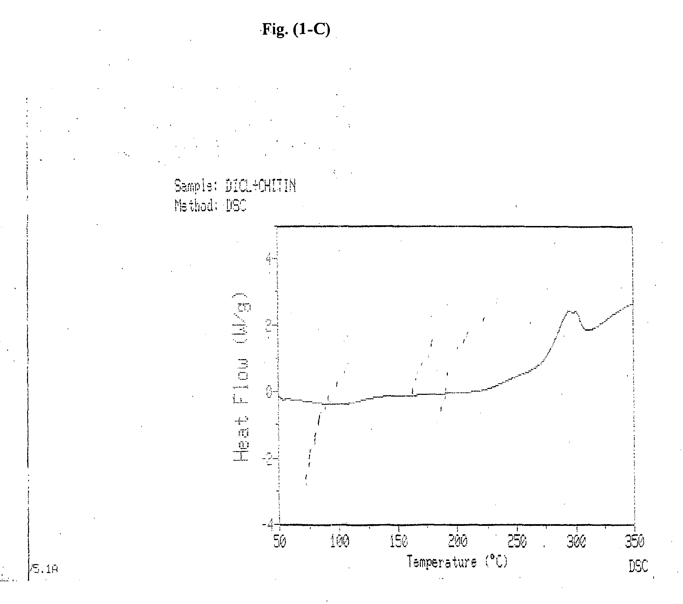

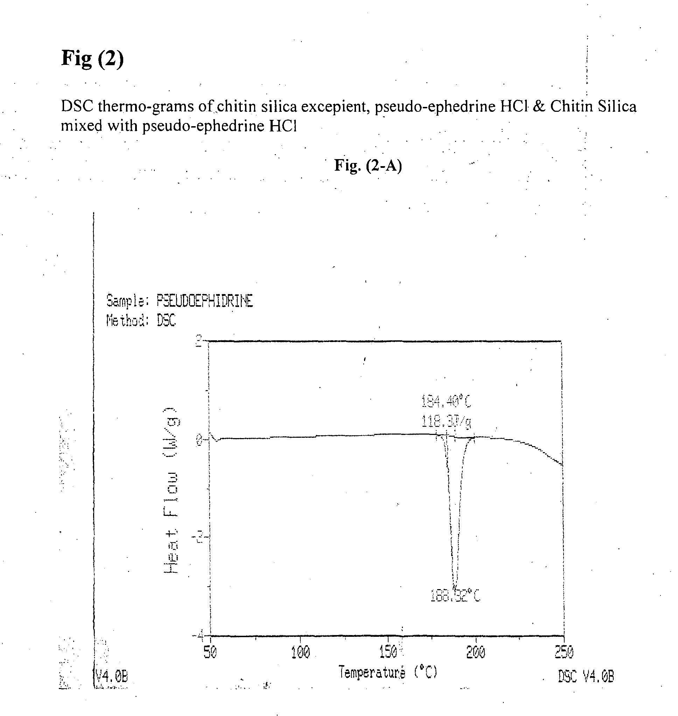

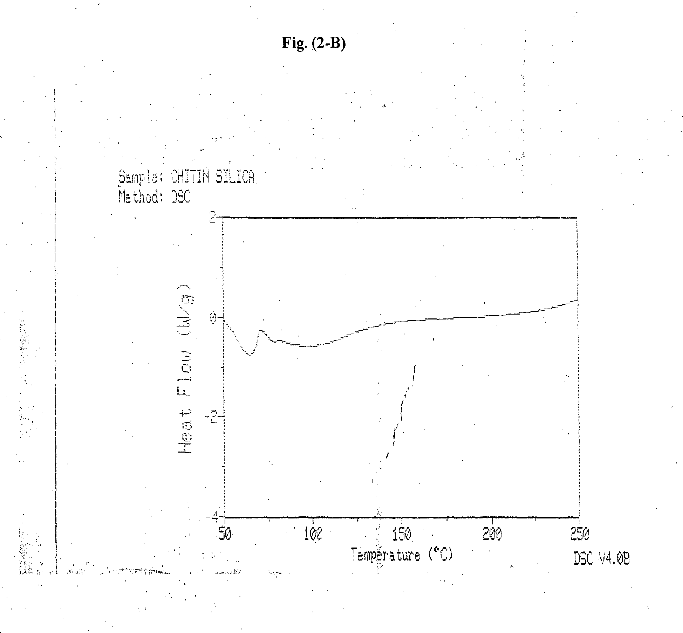

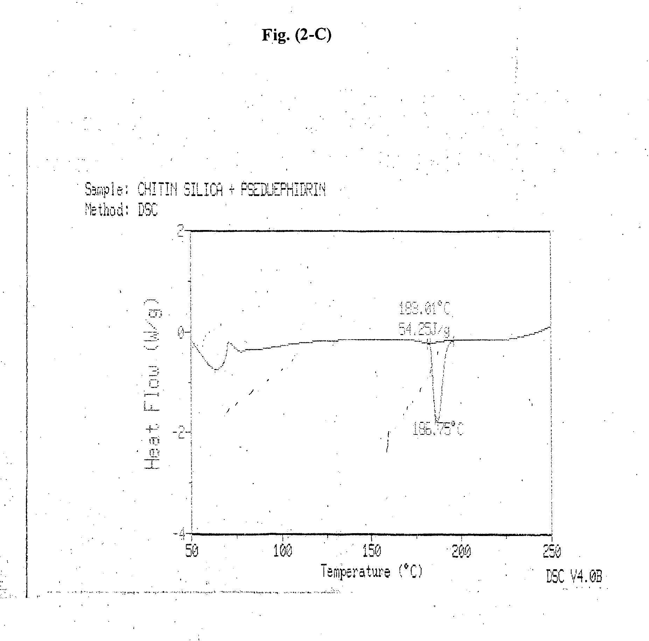

Figures 1A-C shows the DSC thermograms of diclofenac potassium, chitin silica, diclofenac potassium: 1:1 w/w. Figures 2A-C shows the DSC thermograms of pseudoephedrine HCl, chitin silica, and pseudoephedrine HCl:chitin silica 1:1 w/w. DSC thermograms indicate the compatibility of the novel excipient with acidic and basic model drugs can be seen i.e. chitin silica is an inert excipient that does not react with the model acidic or basic molecules.

### Example 5:

### The use of chitin silica as a disintegrant in wet granulation

Metformin 500mg tablet was chosen as a model formula, which undergoes wet granulation during processing. Chitin silica was used as a disintegrant and was granulated either inside (IN) the granulated components, or added after drying and sieving the granulated components (OUT). The formula had the following components and percentages:

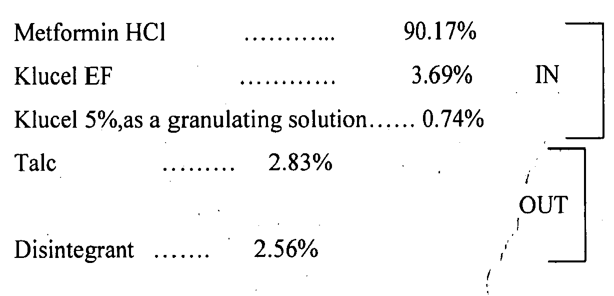

Accordingly the tablet weight was fixed at 554.4 mg in order to contain 500.0mg of the metformin HCl active.
Four formulas were prepared:
F1: The formula contained crosspovidon (a conventional superdisintegrant) added outside the dried granules of Metformin HCl and Klucel.
F2: The formula contained chitin-silica as disintegrant granulated inside the granulated components with metformin and klucel.
F3: The formula contained chitin-silica added outside the dried granules of metformin and klucel.
F4: The formula had no disintegrant. Talc was the only component added outside the dried granules.
The dried powder was passed over sieve US mesh # 22 prior to addition of the disintegrant and lubricant, Then finally, the formula was compressed using punch size 13mm shallow concave at 35 KN, The resulting tablets were investigated according to hardness and disintegration time as well as dissolution.
The dissolution was conducted in 1000 ml USP phosphate buffer pH 6.8. USP dissolution apparatus II, the speed was 50 rpm, and temperature was fixed at 37 °C. Results are shown in Table 5.

**Table 5: Summary of different formulas and their physical characteristics together with dissolution profiles.**

| **Formula** | **Hardness (N)** | **Disintegration Time** | **% Release after 5 minutes Dissolution** | **% Release after 10 minutes Dissolution** |
|---|---|---|---|---|
| F1 | 72 | 1.0min | 87.7% | 99.2% |
| F2 | 77 | 55 Seconds | 101% | 101.5% |
| F3 | 76 | 59 Seconds | 102% | 102.3% |
| F4 | 75 | 8.0min | Not done | Not done |

Results indicate that the superdisintegration power of chitin-silica is not affected by wet granulation. Superior drug release was observed for the chitin silica disintegrant formula compared to crosspovidon disintegrant formula

### Example 6:

### Chitin silica ageing during storage under accelerated conditions

Ageing effect was studied for chitin silica exposed to accelearated stability conditions. The formulas were prepared as mentioned in example 5. Tablets were investigated according to hardness and disintegration time as well as dissolution as previously mentioned in example 5.

**Table 6: Effect of storage of different formulas at 40 °C/75% relative humidity in opened and closed conditions for 2 weeks**

| **Formula** | **Condition** | **Hardness (N)** | **Disintegration Time (seconds)** | **% Release after 5 minutes Dissolution** |
|---|---|---|---|---|
| F1 | 40/75 Closed | 71 | 80 | 82.3 |
| F1 | 40/75 Opened | 65 | 60 | 80.6 |
| F2 | 40/75 Closed | 77 | 60 | 102.1 |
| F2 | 40/75 Opened | 67 | 46 | 100.9 |
| F3 | 40/75 Closed | 70 | 57 | 101.4 |
| F3 | 40/75 Opened | 68 | 47 | 99.8 |

All formulas left under accelerated conditions showed similar physical characteristics and dissolution profiles when compared to initial data.

### Example 7

### Crystallinity versus compactability of chitin silica

To prove the crystallinity x-ray diffraction was carried out for powder chitin, silica and chitin silica coprecipitate

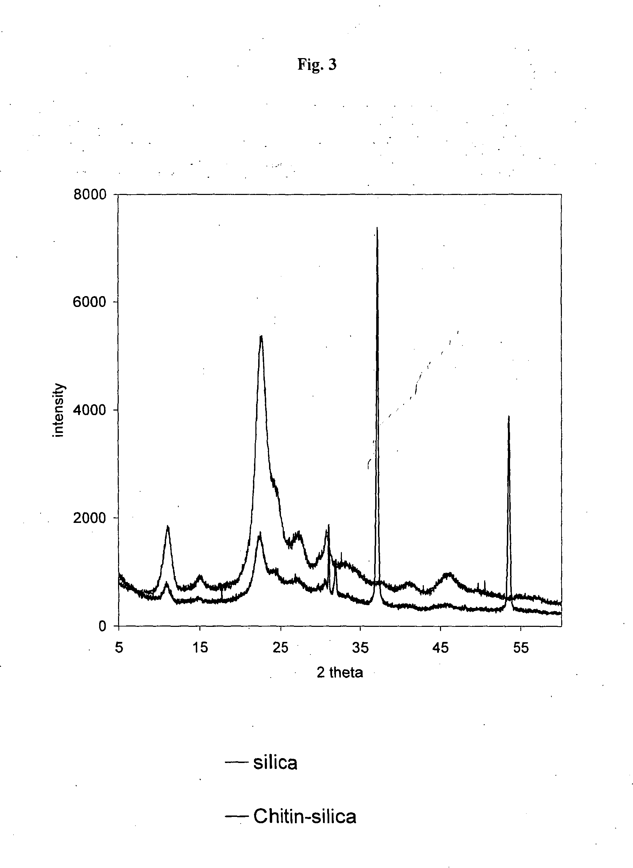

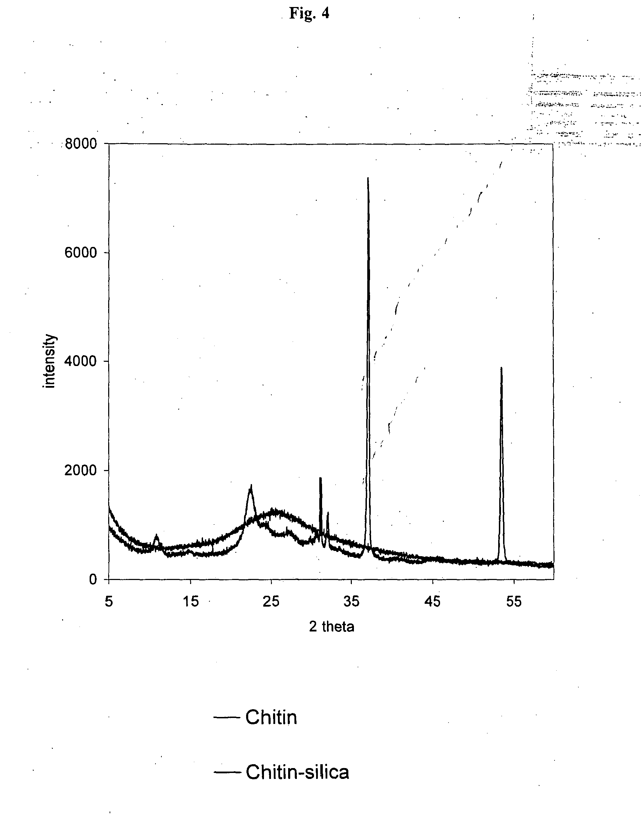

X-ray diffractometer (Philips PW 1729 X-Ray Generator). The XRD patterns were measured with x-ray diffractometer. Radiations generated from Co K_{α} source and filtered through Ni filters with a wavelength of 1,79025 Å at 40 mA and 35 kV were used. The instrument was operated over the 2θ range of 5-65°. Results are shown in Figures 3 and 4. The appearance of new high intensity x ray peaks is a clear indication of crystallinity of chitin silica co-precipitate. The increase in crystallinity may be the cause for the improvement in compactability of the novel excipient.

The features disclosed in the foregoing description, in the claims and/or in the accompanying drawings may, both separately and in any combination thereof, be material for realizing the invention in diverse forms thereof.

## Claims

1. A co-precipitate comprising at least one hydrophilic polymeric compound selected from the group consisting of chitin, chitin derivatives, other polyglucosamines, or any salts, solvates or mixtures thereof, and at least one water-absorbant compound selected from the group consisting of silicon dioxide, derivatives thereof, or any mixtures thereof.

2. The co-precipitate according to claim 1, wherein the hydrophilic polymeric compound is selected from the group consisting of chitin and chitosan.

3. The co-precipitate according to claim 1 or 2, wherein the water-absorbant compound is selected from the group consisting of silicon dioxide and silicates.

4. The co-precipitate according to claim 3, wherein the silicon dioxide is a colloidal silicon dioxide.

5. The co-precipitate according to any of the preceding claims, wherein the amount of the water-absorbant compound in the co-precipitate is in the range of 1 to 75% w/w.

6. The co-precipitate according to claim 5, wherein the amount of the water-absorbant compound in the co-precipitate is about 50% w/w.

7. The co-precipitate according to any of the preceding claims, wherein the hydrophilic polymeric compound has an average degree of deacetylation of 0 to 50%.

8. The co-precipitate according to claim 7, wherein the hydrophilic polymeric compound has an average degree of deacetylation of 0 to 10%.

9. The co-precipitate according to any of the preceding claims, wherein the hydrophilic polymeric compound has a molecular weight ranging from 1.000 to 10.000.000 grams per moles.

10. The co-precipitate according to claim 9, wherein the hydrophilic polymeric compound has a molecular weight ranging from 2.000 to 1.000.000 grams per moles.

11. The co-precipitate according to claim 10, wherein the hydrophilic polymeric compound has a molecular weight ranging from 50.000 to 1.000.000 grams per moles.

12. A method for preparing a co-precipitate according to any of the preceding claims by mixing a wet mass of the hydrophilic polymeric compound with a suspension or solution of the water-absorbant compound in an appropriate ratio and adjusting the process conditions in order to allow the coprecipitate to form.

13. The method according to claim 12, wherein the pH of the mixing solution is between 4 and 10.

14. The method according to claim 13, wherein the pH of the mixing solution is in the range of 4 to 10.

15. The method according to claim 12 to 14, wherein the process for co-precipitation is accomplished by spray drying, freeze drying or simple solution mixing.

16. Use of a compound selected from the group consisting of chitin, chitin derivatives or a co-precipitate according to any of claims 1 to 12 as a filler and/or disintergrant for solid pharmaceutical dosage forms.
